# FASCICULE DE BREVET EUROPEEN

(11) **EP 0 703 203 B1**
(45) Date de publication et mention de la délivrance du brevet: **09.06.1999**
(21) Numéro de dépôt: 95202517.9
(22) Date de dépôt: 18.09.1995
(51) Int. Cl.: C07C 17/087, C07C 19/08, C07C 19/12

(54) **Procédé de fabrication de 1,1,1-trihaloéthanes**
Verfahren zur Herstellung von 1,1,1-Trihaloethanen
Process for the preparation of 1,1,1-trihaloethanes

(30) Priorité: 26.09.1994 FR 9411564
(43) Date de publication de la demande: 27.03.1996
(73) Titulaire: SOLVAY (Société Anonyme), B-1050 Bruxelles (BE)
(72) Inventeur: Balthasart, Dominique, B-1120 Bruxelles (BE); Pennetreau, Pascal, B-1330 Rixensart (BE)
(74) Mandataire: Jacques, Philippe

(56) Documents cités:
- EP-A- 0 391 102
- WO-A-91/18852
- FR-A- 2 124 239
- FR-A- 2 365 542

## Description

La présente invention concerne un procédé de fabrication de 1,1,1-trihaloéthanes, en particulier un procédé de fabrication de 1,1,1-trifluoroéthane, conjointement à du 1,1-dichloro-1-fluoroéthane et/ou du 1-chloro-1,1-difluoroéthane.

Le 1,1,1-trifluoroéthane (ci-après dénommé HFC-143a) apparaît utilisable en particulier comme réfrigérant, en remplacement de chlorofluorocarbures totalement halogénés (CFC), progressivement interdits en raison de leur possible action néfaste à l'encontre de l'ozone stratosphérique.

Le 1,1-dichloro-1-fluoroéthane (ci-après dénommé HCFC-141b) est également utilisable en remplacement de certains CFC, principalement comme agent gonflant de mousses polymériques et comme solvant.

Le 1-chloro-1,1-difluoroéthane (ci-après dénommé HCFC-142b) est également utilisable en remplacement de certains CFC, principalement comme agent gonflant de mousses polymériques. Il est aussi un produit intermédiaire de la synthèse du fluorure de vinylidène.

Il est connu, notamment par le brevet US-2669590, de préparer du HFC-143a par réaction de fluorure de vinylidène (VF2) avec du fluorure d'hydrogène en phase gazeuse, en présence d'un catalyseur. La productivité d'un tel procédé par unité volumique de réacteur est cependant très faible.

Il est par ailleurs connu de préparer du HFC-143a au départ des mêmes réactifs, en phase liquide, à une température de -50 °C, en présence de pentafluorure d'antimoine comme catalyseur (OLAH G.A. et MO Y.K.; Journal of Organic Chemistry, (1972), Vol. 37, No. 7). Le pentafluorure d'antimoine utilisé dans ce procédé est toutefois transformé progressivement en trifluorure d'antimoine inactif en tant que catalyseur et particulièrement corrosif à l'encontre des matériaux métalliques.

Les procédés connus d'hydrofluoration en phase liquide du chlorure de vinylidène (ci-après dénommé VC2) ou du 1,1,1-trichloroéthane (ci-après dénommé T111) ne permettent pas d'obtenir des rendements élevés en HFC-143a. Ces procédés connus produisent en effet essentiellement du HCFC-141b et du HCFC-142b. Dans des conditions telles que celles retenues dans le procédé décrit dans la demande internationale W0 91/18852 où on réalise l'hydrofluoration du VC2 en phase liquide en absence de catalyseur et à une température de 75 à 130 °C, la fraction molaire du HFC-143a formé ne dépasse pas environ 4 % de l'ensemble des produits formés. Dans des conditions favorisant une fluoration plus importante des réactifs halogénés en C2, par exemple à une température plus élevée et en présence de catalyseurs d'hydrofluoration très actifs, tel des sels d'antimoine, du HCFC-142b est principalement formé, le recyclage de celui-ci au réacteur ne permettant pas d'atteindre une productivité en HFC-143a satisfaisante d'un point de vue industriel. En outre, dans ces conditions, on risque de former une quantité importante de sous-produits lourds non souhaités, principalement des oligomères.

La présente invention vise dès lors à fournir un procédé de préparation du HFC-143a qui ne présente plus les inconvénients des procédés mentionnés ci-dessus et qui puisse être facilement mis en oeuvre industriellement.

La présente invention vise également à fournir un procédé de fabrication de HFC-143a et, conjointement, de HCFC-141b et/ou de HCFC-142b, dans lequel les produits recherchés sont formés dans des proportions largement modifiables, en fonction de leurs besoins respectifs.

La présente invention vise également à fournir un procédé permettant d'accroître la proportion du HFC-143a coproduit avec le HCFC-141b et/ou le HCFC-142b dans les procédés existants d'hydrofluoration du VC2 ou du T111 en phase liquide, sans engendrer simultanément la formation de quantités importantes de sous-produits lourds.

L'invention concerne dès lors un procédé de fabrication de 1,1,1-trifluoroéthane (HFC-143a) et, conjointement, de 1,1-dichloro-1-fluoroéthane (HCFC-141b) et/ou de 1-chloro-1,1-dthuoroéthane (HCFC-142b), selon lequel on fait réagir, en phase liquide, du fluorure d'hydrogène (HF), du fluorure de vinylidène (VF2) et au moins un composé chloré choisi parmi le chlorure de vinylidène (VC2) et le 1,1,1-trichloroéthane (T111).

Généralement, on effectue la réaction entre le HF, le VF2 et le composé chloré en phase liquide dans un milieu réactionnel liquide contenant du HCFC-141b. Dans cette forme de réalisation du procédé selon l'invention, le HCFC-141b du milieu réactionnel est avantageusement celui coproduit avec le HFC-143a. La teneur pondérale en HCFC-141b dans le milieu réactionnel est de préférence supérieure à 40 %. De manière particulièrement préférée, elle est de plus de 50 % en poids. Généralement, la teneur en HCFC-141b dans le milieu réactionnel ne dépasse pas 80 %. Outre les réactifs (HF, VC2 ou T111, et VF2) et les produits recherchés de la réaction (HFC-143a, HCFC-141b et HCFC-142b), le milieu réactionnel contient avantageusement également des sous-produits lourds, principalement des oligomères chlorofluorés du VC2. La composition du milieu réactionnel peut être ajustée par une évacuation réglée des produits de la réaction sous deux formes : une évacuation sous forme gazeuse et une évacuation sous forme liquide.

Dans le procédé selon l'invention, la réaction du HF, du VF2 et du composé chloré peut être effectuée en présence ou en absence d'un catalyseur d'hydrofluoration. Dans une forme d'exécution particulière du procédé, la réaction précitée est effectuée en présence d'un catalyseur d'hydrofluoration sélectionné parmi les composés des éléments des groupes IIIa, IVa et b, Va et b et VIb du Tableau périodique des éléments. On retient plus particulièrement les composés de titane, de bore, d'étain, d'antimoine, de tantale et de molybdène. Les composés d'étain ou d'antimoine conviennent très bien, ceux d'étain étant plus particulièrement préférés lorsque le composé chloré est le VC2 et ceux d'antimoine lorsque le composé chloré est le T111. Comme composés utilisables comme catalyseurs, on peut citer les sels et plus particulièrement les halogénures. De préférence, on choisit parmi les chlorures, les fluorures et les chlorofluorures. Le tétrachlorure d'étain s'est révélé particulièrement intéressant. Un composé de formule SnCl₄₋ₓFₓ, avec x compris entre 0 et 4, est tout particulièrement préféré. Un tel composé peut être obtenu par un traitement, de préférence préalablement à la mise en contact avec le VF2 et le composé chloré, du SnCl₄ par du fluorure d'hydrogène. Lorsqu'un catalyseur est utilisé dans le procédé selon l'invention, la quantité de catalyseur mise en oeuvre peut varier dans de larges limites. Habituellement, le catalyseur est utilisé à raison d'au moins 0,001 mole par kg de milieu réactiomel, de préférence, d'au moins 0,01 mole/kg. En général, on n'utilise pas plus d'environ 2 moles de catalyseur par kg de milieu réactionnel. Le plus souvent, on ne dépasse pas environ 1 mole de catalyseur par kg de milieu réactionnel.

Le procédé selon l'invention est généralement effectué à une température de réaction au moins égale à 20 °C. De préférence, la température de réaction est au moins de 40 °C. De manière particulièrement préférée, elle est au moins égale à 60 °C. Habituellement, la température de réaction ne dépasse pas 150 °C. De manière avantageuse, elle ne dépasse pas 130 °C. Une température ne dépassant pas 120 °C est préférée.

La pression à laquelle on exécute la réaction n'est pas critique en elle-même, dès lors qu'elle permet d'effectuer celle-ci en phase liquide, c'est-à-dire qu'elle est suffisante pour maintenir le milieu réactionnel essentiellement sous forme liquide. Elle varie en fonction de la température à laquelle on maintient le milieu réactionnel. Cette pression peut être la pression autogène, une pression supérieure générée par l'introduction d'un gaz inerte, tel que par exemple l'azote, ou une pression inférieure obtenue par dilution du milieu réactionnel avec un solvant organique inerte, tel que par exemple le 1,2-dichloroéthane. Généralement, la réaction est effectuée à une pression au moins égale à 2 bar, de préférence au moins égale à 5 bar. Le plus souvent, la pression ne dépasse pas 30 bar. De manière avantageuse, elle ne dépasse pas 20 bar.

Dans le procédé selon l'invention, la proportion entre la quantité de HFC-143a formé et la quantité de HCFC-141b et/ou de HCFC-142b formés peut être réglée à volonté, par un réglage approprié du rapport molaire du VF2 à la somme du VF2 et du composé chloré mis en oeuvre. En général, le rapport molaire entre le VF2 mis en oeuvre et la somme du VF2 et du composé chloré mis en oeuvre est au moins de 0,01. Le plus souvent, il est au moins égal à 0,05. Généralement, ce rapport molaire ne dépasse pas 0,75. Avantageusement, il ne dépasse pas 0,5.

En absence de catalyseur, le procédé selon l'invention conduit principalement à la formation de HFC-143a et de HCFC-141b, la quantité de HCFC-142b formé étant faible. Par contre, en présence d'un catalyseur, du HFC-143a et du HCFC-142b sont majoritairement formés, la quantité de HCFC-141b formé étant inférieure à celle obtenue en absence de catalyseur.

Dans le procédé selon l'invention, on introduit avantageusement le fluorure d'hydrogène dans un rapport molaire du HF à la somme du VF2 et du composé chloré au moins égal à 1. Le plus souvent, ce rapport molaire ne dépasse pas 20. On travaille de préférence avec un rapport molaire de 1,5 à 15.

Dans le procédé selon l'invention, le HF et le VF2 peuvent être mis en contact préalablement à la mise en contact avec le composé chloré. On peut notamment injecter le VF2 dans du HF non réagi séparé des produits de réaction et qui est recyclé au réacteur d'hydrofluoration du composé chloré. Inversement, le HF et le composé chloré peuvent aussi être mis en contact préalablement à la mise en contact avec le VF2. On peut ainsi par exemple introduire le VF2 au stade de la séparation des produits obtenus par hydrofluoration du composé chloré, notamment par injection dans une colonne de distillation utilisée pour séparer les produits de réaction. De préférence, le HF, le VF2 et le composé chloré sont introduits dans une même zone réactionnelle dans laquelle le HF réagit simultanément avec le VF2 et le composé chloré. Malgré la présence de chlorure d'hydrogène (HCl) dans le milieu réactionnel, généré par la réaction entre le HF et le composé chloré, on a observé que le VF2 réagit très majoritairement avec le HF pour former du HFC-143a plutôt qu'avec le HCl pour former du HCFC-142b.

Dans les conditions décrites ci-dessus, la réaction peut être réalisée en discontinu ou en continu. Elle est avantageusement effectuée en continu.

La réaction peut être réalisée dans un appareillage classique, bien connu de l'homme de l'art. Il peut s'agir de plusieurs réacteurs montés en série ou, de préférence, d'un réacteur unique, alimenté par les matières premières (VC2 ou T111, VF2, HF) et les matières recyclées, sous forme gazeuse ou liquide et chauffé ou refroidi de manière adéquate. Le mode d'introduction ainsi que les débits des réactifs et des recyclages éventuels dans le réacteur sont réglés de façon à maintenir au sein du milieu réactionnel les proportions adéquates conduisant à une production de HFC-143a et, conjointement, de HCFC-141b et/ou de HCFC-142b dans les proportions désirées. Le réacteur est généralement équipé d'un dispositif de soutirage d'un courant gazeux. Le réacteur peut être surmonté par exemple d'une colonne et d'un condenseur de reflux. Ce dispositif permet d'éviter un entraînement, dans le courant gazeux sortant du réacteur, du ou des catalyseurs éventuellement utilisés et d'ajuster la composition de ce courant gazeux. Le réacteur est avantageusement également équipé d'un moyen de soutirage d'un courant liquide. Les moyens de soutirage sont généralement réglés de façon à permettre de soutirer en phase gazeuse essentiellement tout le HCl produit. Lorsqu'une proportion élevée de HCFC-142b est souhaitée, le dispositif de soutirage en phase gazeuse est avantageusement réglé de façon à permettre un soutirage de celui-ci sous forme gazeuse.

Le procédé selon l'invention présente l'avantage appréciable d'une productivité en HFC-143a largement supérieure à celle atteinte par les procédés existants d'hydrofluoration de VC2 ou de T111 en phase liquide. Il permet de convertir à peu de frais les installations existantes de production de HCFC-141b ou de HCFC-142b en unités de production conjointe et modulable de ces produits et de HFC-143a. Selon les conditions opératoires retenues, le procédé selon l'invention permet de varier dans une large gamme la distribution des produits recherchés, c'est-à-dire le HFC-143a, le HCFC-141b et le HCFC-142b.

La figure unique du dessin annexé représente schématiquement un mode de réalisation intéressant du procédé selon l'invention.

L'installation schématisée à la figure comprend un réacteur 1, une voie 2 pour l'admission de HF dans le réacteur 1, une voie 3 pour l'admission d'un mélange de VC2 ou de T111 et de VF2 dans le réacteur 1, une voie 8 pour le soutirage d'un courant liquide du réacteur 1 et une voie 4 pour le soutirage d'un courant gazeux du réacteur. Le réacteur 1 est surmonté d'un condenseur 7 qui est en communication avec la voie 4, avec une voie 6 de recyclage d'un condensat au réacteur 1 et avec une voie 5 d'évacuation d'un gaz.

Selon un premier mode particulier de réalisation du procédé selon l'invention, on met en contact le HF, le VF2 et le composé chloré dans le réacteur 1 en l'absence de tout catalyseur, de préférence à une température de 70 à 110 °C, on règle la température du condenseur de manière à ce que le gaz soutiré par la voie 5 contienne le HFC-143a, le HCFC-142b et l'HCl et on soutire par la voie 8 une partie du milieu réactionnel en phase liquide, dont on sépare le HCFC-141b et le HCFC-142b de manière conventionnelle. Une fraction au moins du HCFC-142b co-produit peut être recyclée au réacteur 1. Dans ces conditions, du HFC-143a et du HCFC-141b sont principalement produits. Ce mode de réalisation du procédé selon l'invention est préféré.

Selon un deuxième mode particulier de réalisation du procédé selon l'invention, on met en contact le HF, le VF2 et le composé chloré dans le réacteur 1, de préférence à une température de 60 à 120 °C, en présence d'un catalyseur d'hydrofluoration, celui-ci étant avantageusement un halogénure d'étain ou d'antimoine, on règle la température du condenseur de manière à ce que le gaz soutiré par la voie 5 contienne principalement du HCFC-141b, du HCFC-142b, du HFC-143a et de l'HCl, dont on sépare le HCFC-141b, le HCFC-142b et le HFC-143a de manière conventionnelle. Une fraction du HCFC-141b co-produit peut être recyclée au réacteur 1. Dans ces conditions, du HFC-143a et du HCFC-142b sont principalement produits.

Les exemples suivants illustrent l'invention de manière non limitative.

### Exemple 1

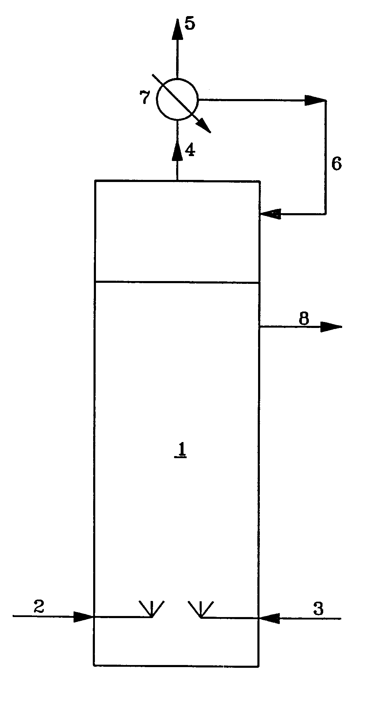

On a injecté de manière continue dans un réacteur tel que schématisé à la figure 1, maintenu à une température de 88 °C et à une pression de 11 bar, contenant un milieu réactionnel liquide constitué principalement de HCFC-141b et contenant 1 g de chlorure d'étain par kilo de milieu réactionnel, du HF, du VC2 et du VF2 dans un rapport molaire 23,5 / 13,9 / 1. Le courant gazeux récupéré à la sortie du condenseur maintenu à 65 °C a été analysé. 99,9 % du VF2 et 99,7 % du VC2 mis en oeuvre ont été transformés. La distribution des produits recherchés, c'est-à-dire le rapport entre la quantité formée de chacun de ces produits et la somme des quantités formées de HCFC-141b, HCFC-142b et HFC-143a, exprimée en pourcentage molaire, était de 39,6 % de HCFC-141b, 54,3 % de HCFC-142b et 6,1 % de HFC-143a.

### Exemple 2 (comparaison)

On a répété l'essai de l'exemple 1 sans introduire de VF2 dans le réacteur. La distribution des produits recherchés était de 41,6 % de HCFC-141b, 57,2 % de HCFC-142b et 1,2 % de HFC-143a.

Sur base des résultats des exemples 1 et 2, on a calculé que, dans le procédé de l'exemple 1 conforme à l'invention, au moins 84 % du VF2 introduit dans le réacteur a été converti en HFC-143a.

## Revendications

1. Procédé de fabrication de 1,1,1-trifluoroéthane et, conjointement, de 1,1-dichloro-1-fluoroéthane et/ou de 1-chloro-1,1-difluoroéthane par réaction en phase liquide de fluorure d'hydrogène, de fluorure de vinylidène et d'au moins un composé chloré choisi parmi le chlorure de vinylidène et le 1,1,1-trichloroéthane.

2. Procédé selon la revendication 1, dans lequel on effectue la réaction dans un milieu liquide contenant de 40 % à 80 % en poids de HCFC-141b.

3. Procédé selon la revendication 1 ou 2, dans lequel on fait réagir le fluorure d'hydrogène simultanément avec le fluorure de vinylidène et avec le composé chloré.

4. Procédé selon une quelconque des revendications 1 à 3, dans lequel on effectue la réaction à une température de 20 à 150 °C.

5. Procédé selon la revendication 4, dans lequel on effectue la réaction à une température de 40 à 130 °C.

6. Procédé selon une quelconque des revendications 1 à 5, dans lequel on effectue la réaction en absence de catalyseur.

7. Procédé selon une quelconque des revendications 1 à 5, dans lequel on effectue la réaction en présence d'un catalyseur d'hydrofluoration sélectionné parmi les composés des éléments des groupes IIIa, IVa et b, Va et b et VIb du Tableau périodique des éléments.

8. Procédé selon une quelconque des revendications 1 à 7, dans lequel le rapport molaire du VF2 à la somme du VF2 et du composé chloré mis en oeuvre est de 0,01 à 0,75.

9. Procédé selon une quelconque des revendications 1 à 8, dans lequel le rapport molaire du fluorure d'hydrogène à la somme du VF2 et du composé chloré mis en oeuvre est de 1 à 20.

10. Procédé selon une quelconque des revendications 1 à 9, dans lequel la réaction est exécutée en continu.

## Claims

1. Process for the manufacture of 1,1,1-trifluoroethane and, in addition, 1,1-dichloro-1-fluoroethane and/or 1-chloro-1,1-difluoroethane, by reaction in the liquid phase of hydrogen fluoride, vinylidene fluoride and at least one chloro compound chosen from vinylidene chloride and 1,1,1-trichloroethane.

2. Process according to Claim 1, in which the reaction is carried out in a liquid medium containing from 40% to 80% by weight of HCFC-141b.

3. Process according to Claim 1 or 2, in which the hydrogen fluoride is reacted simultaneously with the vinylidene fluoride and with the chloro compound.

4. Process according to any one of Claims 1 to 3, in which the reaction is carried out at a temperature of 20 to 150°C.

5. Process according to Claim 4, in which the reaction is carried out at a temperature of 40 to 130°C.

6. Process according to any one of Claims 1 to 5, in which the reaction is carried out in the absence of catalyst.

7. Process according to any one of Claims 1 to 5, in which the reaction is carried out in the presence of a hydrofluorination catalyst selected from compounds of the elements from groups IIIa, IVa and b, Va and b and VIb of the Periodic Table of the Elements.

8. Process according to any one of Claims 1 to 7, in which the molar ratio of the VF2 to the sum of the VF2 and chloro compound used is from 0.01 to 0.75.

9. Process according to any one of Claims 1 to 8, in which the molar ratio of the hydrogen fluoride to the sum of the VF2 and chloro compound used is from 1 to 20.

10. Process according to any one of Claims 1 to 9, in which the reaction is conducted continuously.

## Patentansprüche

1. Verfahren zur Herstellung von 1,1,1 -Trifluorethan und, damit zusammen, von 1,1-Dichlor-1-fluorethan und/oder von 1-Chlor-1,1-difluorethan durch Reaktion in flüssiger Phase von Fluorwasserstoff, Vinylidenfluorid und wenigstens einer chlorierten Verbindung, die unter Vinylidenchlorid und 1,1,1-Trichlorethan ausgewählt ist.

2. Verfahren gemäß Anspruch 1, bei dem man die Reaktion in einem flüssigen Medium ausführt, das 40 bis 80 Gew.-% HCFC-141b enthält.

3. Verfahren gemäß Anspruch 1 oder 2, bei dem man Fluorwasserstoff gleichzeitig mit dem Vinylidenfluorid und mit der chlorierten Verbindung reagieren läßt.

4. Verfahren gemäß einem der Ansprüche 1 bis 3, bei dem man die Reaktion bei einer Temperatur von 20 bis 150 °C ausführt.

5. Verfahren gemäß Anspruch 4, bei dem man die Reaktion bei einer Temperatur von 40 bis 130 °C ausführt.

6. Verfahren gemäß einem der Ansprüche 1 bis 5, bei dem man die Reaktion in Abwesenheit von Katalysator ausführt.

7. Verfahren gemäß einem der Ansprüche 1 bis 5, bei dem man die Reaktion in Gegenwart eines Katalysators zur Hydrofluorierung ausführt, der unter den Verbindungen der Elemente der Gruppen IIIa, IVa und b, Va und b und VIb des Periodensystems der Elemente ausgewählt ist.

8. Verfahren gemäß einem der Ansprüche 1 bis 7, bei dem das Molverhältnis von VF2 zur Summe von VF2 und der chlorierten Verbindung, die eingesetzt wird 0,01 bis 0,75 beträgt.

9. Verfahren gemäß einem der Ansprüche 1 bis 8, bei dem das Molverhältnis von Fluorwasserstoff zur Summe von VF2 und der chlorierten Verbindung, die eingesetzt wird, 1 bis 20 beträgt.

10. Verfahren gemäß einem der Ansprüche 1 bis 9, bei dem die Reaktion kontinuierlich ausgeführt wird.
